# EUROPEAN PATENT SPECIFICATION

(11) **EP 2 197 380 B1**
(45) Date of publication and mention of the grant of the patent: **12.02.2014**
(21) Application number: 08832667.3
(22) Date of filing: 17.09.2008
(51) Int. Cl.: A61B 18/18

(54) **ELECTROSURGICAL INSTRUMENT AND METHOD**
ELEKTROCHIRURGISCHES INSTRUMENT UND VERFAHREN
INSTRUMENT ET PROCÉDÉ ÉLECTRO-CHIRURGICAL

(30) Priority: 18.09.2007 US 973254 P; 18.03.2008 US 50462
(43) Date of publication of application: 23.06.2010
(73) Proprietor: Ethicon Endo-Surgery, Inc., Cincinnati, OH 45242 (US)
(72) Inventor: BAKER, John, Palo Alto California 94306 (US)
(74) Representative: Tunstall, Christopher Stephen
(86) International application number: PCT/US2008/076683
(87) International publication number: WO 2009/039179

(56) References cited:
- US-A- 4 492 231
- US-A- 5 318 565
- US-A- 6 113 598
- US-A1- 2002 107 517
- US-A1- 2004 116 979
- US-A1- 2005 203 507
- US-B2- 6 808 525
- US-B2- 7 112 201

## Description

### BACKGROUND OF THE INVENTION

### 1. Field of the Invention

The present description relates to medical devices and methods. More particularly, the present description relates to electrosurgical instruments, working ends, and methods for sealing and transecting tissue.

### 2. Description of the Related Art

In various open and laparoscopic surgeries, it is necessary to coagulate, seal or fuse tissues. One preferred means of tissue-sealing relies upon the application of electrical energy to captured tissue to cause thermal effects therein for sealing purposes. Various mono-polar and bi-polar radiofrequency (Rf) jaw structures have been developed for such purposes. In general, the delivery of Rf energy to a captured tissue volume elevates the tissue temperature and thereby at least partially denatures proteins in the tissue. Such proteins, including collagen, are denatured into a proteinaceous amalgam that intermixes and fuses together as the proteins renature. As the treated region heals over time, this biological "weld" is reabsorbed by the body's wound healing process.

In a typical arrangement of a bi-polar radiofrequency (Rf) jaw, the face of each jaw comprises an electrode. Rf current flows across the captured tissue between electrodes in opposing jaws. Most commercially available bi-polar jaws provide a low tissue strength weld immediately post-treatment. While they can adequately seal or weld tissue volumes having a small cross-section, such bi-polar instruments often are ineffective at sealing or welding many types of tissues, such as anatomic structures having walls with irregular or thick fibrous content, bundles of disparate anatomic structures, substantially thick anatomic structures, or tissues with thick fascia layers such as large diameter blood vessels. Additionally, many important surgical applications, particularly vessel transection procedures, relate to sealing blood vessels which contain considerable fluid pressure therein. Such applications require a high strength tissue weld immediately post-treatment not provided by currently available Rf jaws.

Moreover, currently available Rf jaws that engage opposing sides of a tissue volume typically cannot cause uniform thermal effects in the tissue, whether the captured tissue is thin or substantially thick. As Rf energy density in tissue increases, the tissue surface becomes desiccated and resistant to additional ohmic heating. Localized tissue desiccation and charring can occur almost instantly as tissue impedance rises, which then can result in a non-uniform seal in the tissue. Typical currently available Rfjaws can cause further undesirable effects by propagating Rf density laterally from the engaged tissue to cause unwanted collateral thermal damage. US patent publication no. 2004/0116979 relates to an electrosurgical medical device and method for creating thermal welds in engaged tissue. In one embodiment, at least one jaw of the instrument defines a tissue engagement plane that carries a recessed central portion. In another embodiment, the controller coupled to the Rf source is adapted to switch from a power control operational mode to a voltage controlled operational mode at a selected transition impedance level. US patent no. 4, 492,231 relates to a non-sticking electrocautery system and forceps

Therefore, there is a need for surgical instruments and working ends which avoid at least some of the shortcomings of present devices for sealing and transecting tissue structures.

### BRIEF SUMMARY OF THE INVENTION

The invention is set out in the appended claims. The embodiments of the description which do not fall within the scope of the claims are provided for illustrative purposes only and do not form part of the present invention.

The object of the present invention is to provide a working end of a surgical instrument capable of transecting and compressing tissue. The working end allows for controlled Rf energy delivery to transected tissue margins having thick fascia layers, or other tissue layers with non-uniform fibrous content.

In a first aspect, embodiments of the present description provide a method for delivering energy to a selected tissue structure, preferably to controllably seal the tissue. The tissue is progressively clamped between a set of jaws, for example the first and second jaws of a working end of an electrosurgical instrument. Fibrous tissue layers (*i.e*., fascia) conduct radiofrequency (Rf) current differently than adjacent less-fibrous tissue layers. Differences in extracellular fluid content in such adjacent tissues contribute greatly to the differences in ohmic heating. By applying high compressive forces to the tissue layers, extracellular fluids migrate from the site to collateral regions, thereby making electrical resistance much more uniform regionally within the selected volume of tissue.

Each of the jaws may comprise an energy delivery surface. The jaws are preferably adapted to open and close relative to each other with a selectable degree of jaw closure between the first and second jaw. Electrosurgical energy, typically radiofrequency (Rf) energy, is applied to the volume of tissue through the energy delivery surfaces, typically opposing polarity surfaces, in the jaws. Electrosurgical energy is applied in either a first mode or a second mode. The application of electrosurgical energy in the first mode may be configured to substantially dehydrate the tissue and reduce its cross-section. The applipation of electrical surgical energy in the second mode may be configured to weld the tissue. In many embodiments, the application of electrosurgical energy may be switched from the first mode to the second mode in response to a measured, operational parameter. The parameter may be, for example, the rate of jaw closure, the degree of jaw closure, the impedance of the tissue, or a given time interval. The jaws of the invention may operate in mono-polar or bi-polar modalities.

The jaws may include a resistive heating element and may carry a core conductive material or electrode coupled to an Rf source and controller. The material may comprise a fixed resistance material, a material having a positive temperature coefficient of resistance (PTCR) or a material having a negative temperature coefficient of resistance (NTCR). A PTCR material may be engineered to exhibit a dramatically increasing resistance above a specific temperature of the material, sometimes referred to as a Curie point or a switching range. In embodiments comprising a PTCR material, when the tissue temperature elevates the temperature of the PTCR material to the switching range, Rf current flow from jaws will be terminated. The instant and automatic reduction of Rf energy application may prevent any substantial dehydration of tissue clasped by the jaws.

In many embodiments, energy may be applied in the first mode through selected portions of the first and second jaws. In the second mode, energy may be applied through different selected portions of the first and second jaws. The selected portions in the first mode may be the peripheral portions of the jaws and the selected portions in the second mode may be the non-peripheral portions of the jaws.

Alternatively, the first mode of delivery energy may comprise the delivery of radiofrequency (Rf) energy and the second mode of delivering energy may comprise heat conduction. Electrosurgical-energy may be applied to the tissues by Rf ohmic heating from bi-polar electrodes in one or both jaws. When Rf ohmic heating is limited by impedance, energy may instead be applied through heat conduction from a resistive heating material in one or both jaws.

In another aspect, embodiments of the present description provide an electrosurgical instrument comprising an instrument body, a working end on the instrument body, and a control system. The working end is an openable-closeable jaw structure with a first and second jaw, each with energy delivery surfaces. The working end has a degree of closure between 0% and 100%. The control system may be configured to activate the energy delivery surfaces in a first mode, a second mode, or both based on an operational parameter. Energy delivery in the first mode may be configured to substantially dehydrates and reduces the cross-section of the tissue. Energy delivery in the second mode may be configured to weld the tissue.

In many embodiments, the control system switches between activating the energy delivery surfaces in the first mode and activating the energy delivery surfaces in the second mode in response to a change in the operational parameter. The operational parameter may be, for example a degree of jaw closure, a rate of jaw closure, an impedance of the tissue, or a time interval.

In many embodiments, a portion of the energy delivery surfaces of the jaws may comprise a resistive heating element, for example a resistive heating material. Another portion may comprise an Rf element. The resistive heating element delivers heat to the tissue when Rf paths are limited due to increased tissue impedance. The resistive heating material may extend over at least 5% of the energy delivery surface.

In many embodiments, the jaws may comprise a positive temperature coefficient of resistance (PTCR) material or a negative temperature coefficient of resistance (NTCR) material.

In many embodiments, each of the energy delivery surfaces comprises at least one radiofrequency electrode. The electrodes may be arranged to be connected to opposite poles of a bipolar power supply in the control system. In some embodiments, the control system in the first mode activates the electrodes in the surfaces of the first and second jaws. In the second mode, the control system also activates the electrodes in the surfaces of the first and second jaws but with the activated electrodes having a polarity opposite of that of the first mode. In some embodiments, the control system in the first mode activates electrodes in the surfaces of the first and second jaws and the in the second mode, activates electrodes within a surface of at least one of the jaws.

In many embodiments, the instrument body comprises an axially reciprocating member. The axially reciprocating member may be carried by the instrument body and may be configured to open and close the jaws. Axial movement of the reciprocating member is configured to switch the activation of the electrosurgical surfaces from the first mode to the second mode. Axial movement of the reciprocating member may also be configured to transect tissue clasped by the jaws, for example by having the reciprocating member comprise a tissue-cutting element, such as a sharp distal edge.

In some embodiments, the first jaw and second jaw each comprise a plurality of electrodes and the control system may be configured to activate different sets of electrodes based on at least one of the percentage of jaw closure and the impedance of tissue captured between the first and second jaw.

In many embodiments, the electrosurgical surfaces of the first jaw and the second jaw each include a resistive heating element. The surfaces may also include an active electrode and a resistive material comprising a percentage of the surface. The resistive material may be an PTCR material, an NTCR material, or material having a fixed resistance. In some embodiments, the electrosurgical surfaces comprise bi-polar electrodes. In the first mode, the bi-polar electrodes in the first and second jaws may be activated. In the second mode, the bi-polar electrodes within the surfaces of at least one of the jaws may be activated and the bi-polar electrodes in surfaces of the first and second jaws may be activated as well.

Alternatively, the first jaw and second jaw comprise a plurality of opposing polarity electrodes. The control system may be configured to activate different sets of opposing polarity electrodes based on the percentage of jaw closure and/or the impedance of tissue captured between the first and second jaw.

Another aspect of the description provides means for creating high compression forces along the very elongate working end of a electrosurgical instrument that engages a volume of targeted tissue. A slidable or translatable extension member is provided. The extension member may define cam surfaces that engage the entire length of jaw members as the extension member is extended over the jaws. The extension member may be adapted to perform multiple functions including but not limited to contemporaneously closing the jaws of a working end and transecting the engaged tissue, applying very high compression to the engaged tissue, and cooperating with electrosurgical components of the jaws to deliver thermal energy to the engaged tissue.

The combination of the extension member in cooperation with the jaws of the working end thus allows for electrosurgical electrode arrangements that are adapted for controlled application of current to engaged tissue. An electrosurgical instrument according to embodiments of the present invention comprises an openable-closeable jaw assembly with first and second iaw members comprising electrosurgical energy-delivery surfaces. Each jaw member comprise an opposing polarity conductive body coupled to an electrical source. At least one jaw surface comprises a partially resistive body. The partially resistive body may have a fixed resistance, a resistance that changes in response to pressure, or a resistance that changes in response to temperature. The partially resistive body is capable of load-carrying to prevent arcing in tissue about the energy-delivery surfaces to create and effective weld without charring or desiccation of tissue.

In many embodiments, the working end comprises components of a sensor system which together with a power controller can control Rf energy delivery during a tissue welding procedure. For example, feedback circuitry for measuring temperatures at one or more temperature sensors in the working end may be provided. Another type of feedback circuitry may be provided for measuring the impedance of tissue engaged between various active electrodes carried by the working end. The power controller may continuously modulate and control Rf energy delivery in order to achieve (or maintain) a particular parameter such as a particular temperature in tissue, an average of temperatures measured among multiple sensors, a temperature profile (change in energy delivery over time), or a particular impedance level or range.

Another aspect of the present description provides a medical instrument comprising a shaft with a working end, a handle end coupled to the shaft, an articulating structure within the working end, and an actuator mechanism in the handle end. The working end comprises a pair of openable-closeable jaws. The handle end may be rotatable relative to the shaft. The articulating structure may articulate the jaws between a non-deflected configuration and a deflected configuration. The actuator mechanism may selectively rotate the shaft and/or articulate the jaws between the two configurations. The actuator mechanism may also comprise a rotatable member which actuates pull wires or may be configured for digital engagement, for example by providing a thumb-wheel. The actuator mechanism may also comprise a switch mechanism that switches the actuation of the rotatable member between rotating the shaft and articulating the jaws between the non-deflected and deflected configurations. For example, the switch mechanism may be a cam-type brake mechanism or a locking mechanism. The actuator mechanism may also be coupled to a motor drive.

In another aspect, embodiments of the present invention provide a medical instrument comprising a shaft having a working end, the working end having a pair of openable-closeable jaws, a handle end coupled to the shaft, a jaw-closing mechanism, a shaft-rotating mechanism, and an articulating mechanism. The jaw-closing mechanism comprises an extension member slidable from a retracted position to an extended position in a longitudinal channel within the jaws for closing the jaws. The extension member have first surfaces that engage cooperating second surfaces of the jaws to move the jaws from an open position toward a closed position. The shaft-rotating mechanism rotates the shaft and working end relative to the handle end. The articulating mechanism articulates the working end between a non-deflected and a deflected configuration. In many embodiments, the articulating mechanism comprises translatable cables.

In many embodiments, the handle end may be coupled to a robotic actuator for actuating the jaw-closing mechanism, actuating the shaft-rotating mechanism, and/or actuating the articulating mechanism. Alternatively, the handle end may be coupled to a computer-controlled drive system. In many embodiments, the jaws comprise energy-delivery surfaces for applying energy to tissue, at least one electrode, bi-polar electrodes, a material having a positive temperature coefficient of resistance material or a resistively heated element. In many embodiments, the handle end comprises a plurality of keyed rotating members for actuating at least one of the jaw-closing mechanism, the shaft-rotating mechanism and the articulating mechanism.

### BRIEF DESCRIPTION OF THE DRAWINGS

FIG. 1 is a perspective view of an electrosurgical device.
FIG. 2A shows a working end of an electrosurgical instrument having a translatable extension member in a non-extended position.
FIG. 2B shows the working end of FIG. 2A with the translatable extension member in an extended position..
FIG. 2C is a sectional view of a translatable member shaped like an I-beam.
FIGS. 3A-3B show a working end of an electrosurgical instrument in a fully open position.
FIG. 4 shows a working end of an electrosurgical instrument in an intermediate closed position.
FIG. 5 is an exploded view of a working end of an electrosurgical instrument in a fully closed position.
FIGS. 6A-6C are sectional views of a working end of an electrosurgical instrument in different modes of operation.
FIG. 7A is a side view of a working end of an electrosurgical instrument carrying a pivot allowing articulation of the jaw structure.
FIG. 7B is a perspective view of a working end carrying at a pivot and a cable to allow articulation of the jaw structure.
FIG. 8A is a perspective view of a handle end of an electrosurgical instrument carrying the working end of FIGS. 7A-B.
FIGS. 8B-C show the handle end of FIG. 8A from different angles.
FIG. 8D is a cut-away view of the handle end of FIG. 8A showing a thumb-wheel.

### DETAILED DESCRIPTION OF THE INVENTION

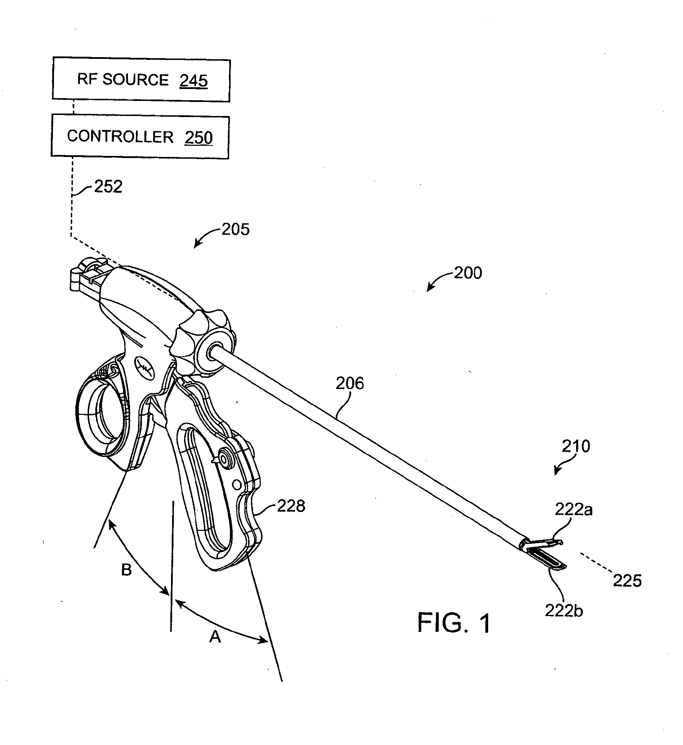

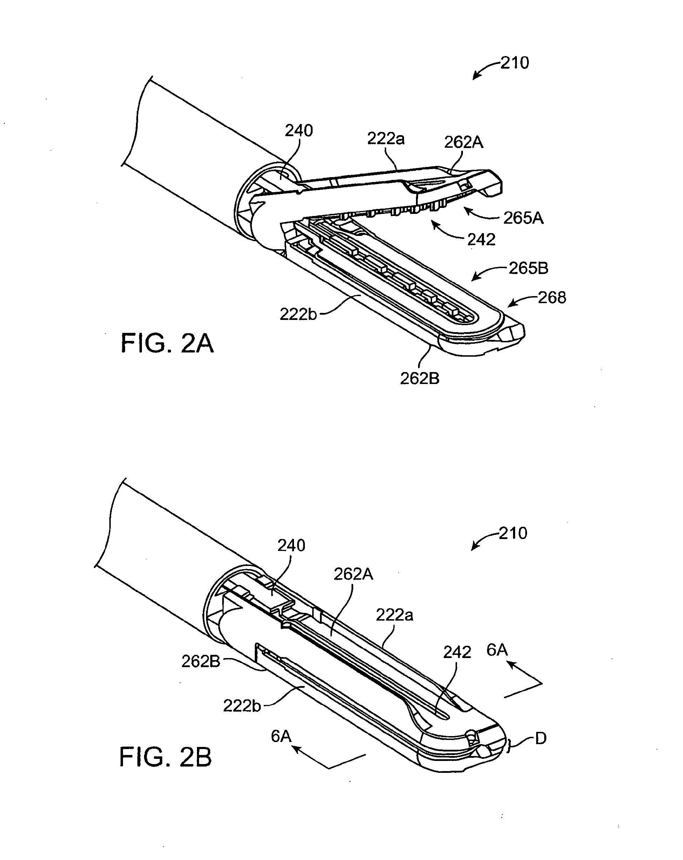

1. Type "A" system for tissue sealing and transection. FIG. 1 shows an electrosurgical instrument **200** with a handle end **205** and introducer or shaft member **206.** Introducer **206** carries the working end **210** and may be adapted for welding and transecting tissue. Working end **210** comprises an openable-closeable jaw assembly with straight or curved jaws, first jaw **222a** and second jaw **222b.** The jaws **222a** and **222b** may close and capture or engage tissue about an axis **225** and may also apply compression to the tissue. Introducer **206** has a cylindrical or rectangular cross-section and can comprises a thin-wall tubular sleeve that extends from handle **205.** Handle **205** comprises a lever arm **228** adapted to actuate a translatable, reciprocating member **240** that also functions as a jaw-closing mechanism. The distal end of reciprocating member **240** comprises a flanged "I"-beam configured to slide within a channel **242** in the jaws **222a** and **222b** as seen in FIGS 2A-2C. Jaw closing mechanisms and electrosurgical energy-delivery surfaces are described in the following US Patents: 7,220,951; 7,189,233; 7,186,253; 7,125,409; 7,112,201; 7,087,054; 7,083,619; 7,070,597; 7,041,102; 7,011,657; 6,929,644; 6,926,716; 6,905,497; 6,802,843; 6,770,072; 6,656,177; 6,533,784; 6,500,176. In embodiments shown by FIG. 1, each jaw member **222a** and **222b** is coupled to electrical source **245** and controller **250** by electrical leads in cable **252** to function as paired bi-polar electrodes with positive polarity (+) and negative polarity (-) as will be further described below.

Handle **205** comprises a moveable lever **228** for closing the jaws. The system and instrument **200** may be configured to provide different electrosurgical energy-delivery modes which may depend on the degree of jaw closure. The degree of jaw closure may be represented by the degree of actuation of lever **228,** for example degrees of actuation **A** and **B** in FIG. 1. Alternatively, the degree of actuation may be represented by the axial translation of reciprocating member **240.** It may be useful to switch between different electrosurgical energy-delivery modes depending on the volume of tissue captured and the degree of compression applied to the tissue. For example, the system and instrument **200** may deliver Rf energy in a first mode to large volumes of engaged tissue to cause initial dehydration. The system and instrument **200** may thereafter switch to a second mode which allows for more effective tissue welding. Alternatively, when engaging a lesser volume of tissue, the system and instrument **200** may deliver Rf energy in the second mode only which is best suited for tissue welding.

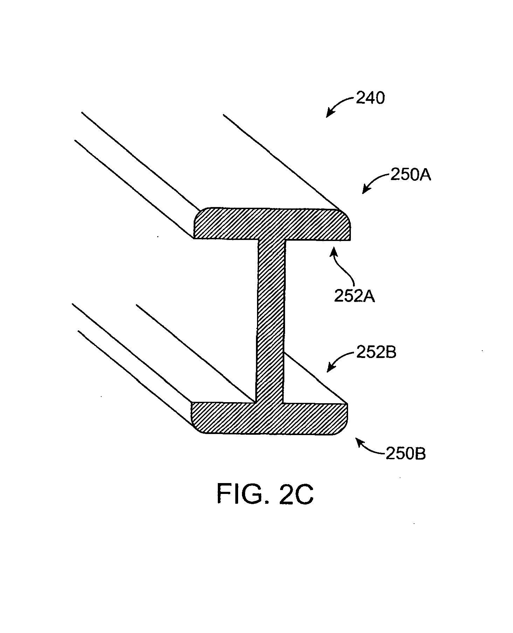

FIG. 2C shows the distal end of reciprocating member **240** having upper and lower flanges or "c"-shaped portions **250a** and **250b.** The flanges **250a** and **250b** respectively define inner cam surfaces **252a** and **252b** for slidably engaging outward-facing surfaces **262a-262b** of jaws **222a** and **222b.** The inner cam surfaces **252a** and **252b** can have any suitable profile to slidably cooperate with surfaces **262a-262b** of jaws **222a** and **222b.** As seen in FIG. 2A-2B, jaws **222a** and **222b** in a closed position define a gap or dimension **D** between the energy-delivery surfaces **265A** and **265B** of jaws **222a** and **222b.** Dimension **D** equals from about 0,0127 mm (0.0005") to about 0,127 mm (0.005") and preferably between about 0,0254 mm (0.001") about 0,0508 mm (0.002"). The edges **268** of energy-delivery surfaces **265a** and **265b** may be rounded to prevent the dissection of tissue. The channel **242** within the jaws accommodates the movement of reciprocating member **240,** which may comprise a tissue-cutting element, for example by having a sharp distal edge.

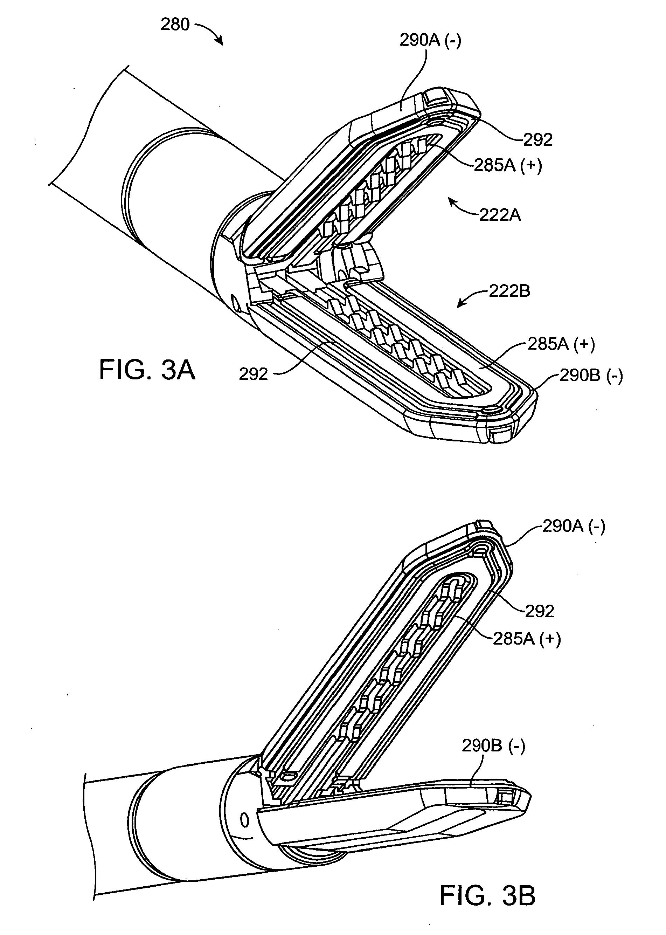

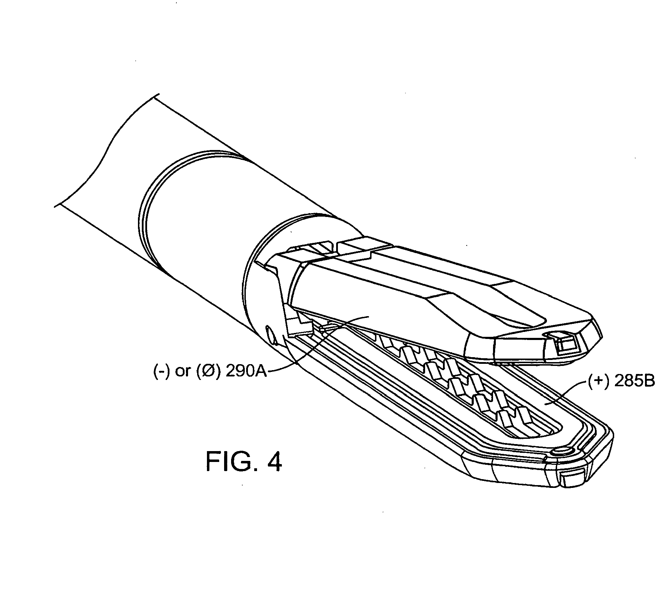

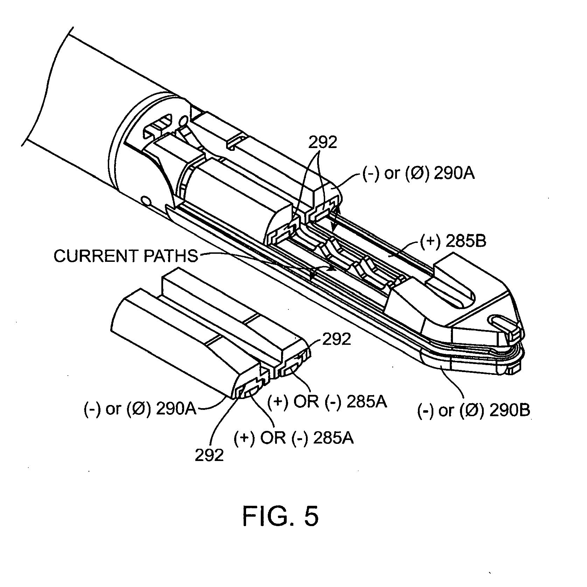

FIGS. 3A and 3B illustrate views of a working end **280** similar to those shown by FIGS. 2A-2B. FIGS. 3A-B show the energy-delivery surfaces of the jaws in more detail. In the embodiments shown, the electrosurgical energy-delivery surfaces are mirror images of one another. The energy-delivery surfaces comprise surface portions of first and second conductive bodies **285A** and **285B** in the interior surface portions of the respective jaws **222a** and **222b.** The embodiments shown further comprise third and fourth mirror-image conductive bodies **290A** and **290B** which are structural, perimeter components of the respective jaws **222a** and **222b.** The embodiments shown further comprise at least one intermediate material **292** intermediate to the first and third conductive bodies **285A** and **290A** in the first jaw **222a.** Intermediate material **292** may also be intermediate to the second and fourth conductive bodies **285B** and **290B** in the second jaw **222b.** The intermediate material **292** may be at least one of an insulator, a positive temperature coefficient of resistance (PTCR) material, or a fixed resistive material. In FIGS. 3A-6C, the first, second, third and fourth conductive bodies **285A, 285B, 290A** and **290B** are indicated in various modes of operation as having polarities indicated as positive polarity (+),negative polarity (-), or an absence of polarity (Ø). The first and second conductive bodies **285A** and **285B** are coupled by electrical leads to Rf source **240** and controller **250** with switching means for switching polarities as described below. In some embodiments, the translatable member **240** can carry electrical current or be coated with an insulator layer to prevent the member **240** from functioning as a conductive path for current delivery. FIGS. 3A and 3B illustrate the first and second jaws **222a** and **222b** a fully open position, FIG. 4 illustrates the jaws **222a** and **222b** in an intermediate closed position, and FIG. 5 illustrates the jaws in a fully closed position in a cut-away view.

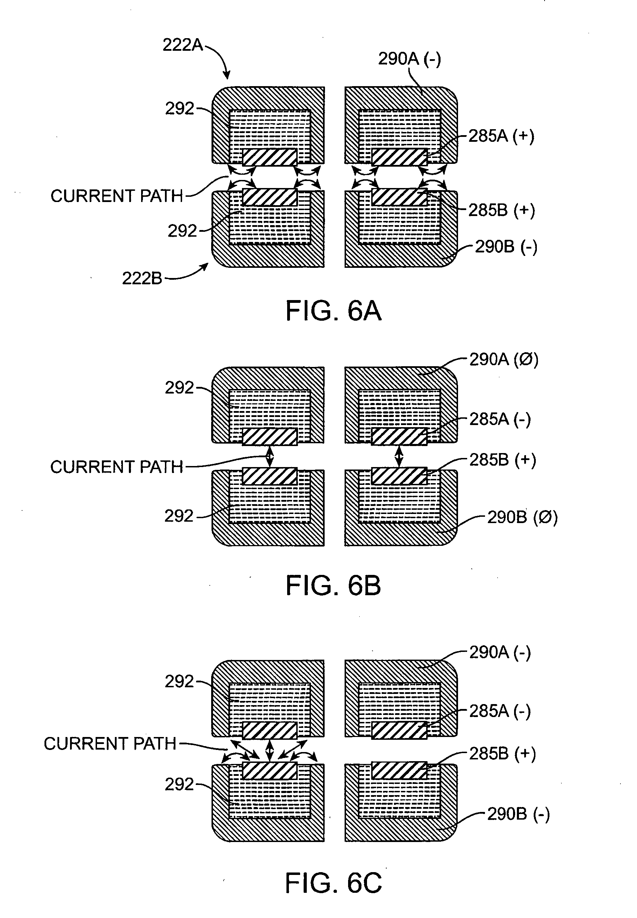

In embodiments shown by FIGS. 3A-5, at least one jaw has the potential of multiple operating modes wherein the polarity of conductive bodies or electrodes **(285A** or **285B)** is switched depending on the degree of jaw closure. Multiple modes are illustrated by the schematic sectional views of FIGS. 6A-6C. In one aspect of the invention, the system **200** and working end **280** are used to practice a method of the invention comprising: (i) providing an electrosurgical working end having openable-closeable paired jaws; (ii) progressively clamping a selected tissue volume between the first and second jaws; and (iii) applying electrosurgical energy to the tissue in either a first mode or a second mode based on the degree of jaw closure.

In one method, a mode of operation comprises substantial energy delivery via Rf current paths between opposing polarity electrodes within a single jaw's energy-delivery surfaces **265a** or **265b.** For example, as can be seen in FIG. 6A, the current paths can be between surface electrodes **285A** (+) and **290A** (-) in the first jaw **222a** and between surface electrodes **285B** (+) and **290B** (-) in the second jaw **222b.** This Rf energy deliver mode is suited for sealing or welding thin or highly compressed tissues volumes.

In this method, another mode of operation comprises switching to substantial energy delivery via Rf current paths between the energy-delivery surfaces **265a** and **265b** of the first and second jaws **222a** and **222b.** For example, as can be seen in FIG. 6B, the interior surface electrodes **285A** (-) and **285B** (+) are switched to have opposing polarities for providing Rf current paths through the engaged tissue to cause dehydration of thick tissue volumes. The second and fourth conductive bodies **290A** and **290B** in the respective first and second jaws **222a** and **222b** may have a null polarity or absence of polarity (Ø).

The operational mode of FIG. 6B wherein Rf current paths are directed between the energy-delivery surfaces **265a** and **265b** of the opposing jaws is useful for dehydrating thick tissue bundles upon engaging and clamping tissue, for example when the jaws are moved from a fully open position or 0% jaw closure (FIGS. 3A-3B) toward a more complete closure. Upon moving the jaws to an intermediate closure (FIG. 4), the operational mode of FIG. 6A provides substantial energy delivery via Rf current paths between opposing polarity electrodes in a single jaw **222a** or **222b** and providing optimal energy delivery for creating a high-strength seal or weld in the engaged tissue.

In one method of the invention, a control system and/or controller **250** switches from one mode to another mode after jaw closure of 10%, 20%, 30%, 40%, 50%, 60%, 70%, 80%. The initial operational mode provides substantial Rf current paths between the energy-delivery surfaces **265a** and **265b** of the opposing jaws (FIG. 6B). The subsequent operational mode provides substantial Rf current paths between opposing polarity electrodes in a single jaw **222a** or **222b** (FIG. 6A). FIG. 6C illustrates another operational mode similar to the mode of FIG. 6A wherein the perimeter conductive bodies **290A** and **290B** are conductive with a (-) polarity.

In one embodiment, the apparatus comprises an electrosurgical instrument with a working end having openable-closeable first and second jaws characterized in operation between 0% and 100% jaw closure, electrosurgical surfaces in the first and second jaws; and a control system configured for activation of the electrosurgical surfaces in first and second modes based on percentage of jaw closure. The control system is configured for activation and switching from the first mode to the second mode after jaw closure of 10%, 20%, 30%, 40%, 50%, 60%, 70%, 80%.

It should be appreciated that the switching between electrosurgical modes can be triggered by one or more operational parameters, such as (i) degree of jaw closure as described above, (ii) impedance of the engaged tissue, (iii) rate of change of impedance or any combination thereof. Further, the scope of the invention includes switching multiple times between various electrosurgical modes during initial tissue engagement, tissue clamping and tissue transection.

In another method, when in an initial engagement with thick tissue, it is useful to deliver energy in the mode of FIG. 6A which dehydrates tissue on both surfaces to prevent subsequent stray Rf current flow into outwardly lying tissue thus helping to prevent collateral tissue damage. After an initial energy delivery interval, which can be determined by at least one of (i) time, (ii) degree of jaw closure or (iii) any impedance parameter-the system can then switch to the energy delivery mode of FIG. 6B. Thereafter, the system can switch to the mode of either FIG. 6A or 6C upon a selected degree of jaw closure, an impedance parameter or a combination thereof.

The electrosurgical method of the invention includes comprises a first mode that activates bi-polar electrodes in surfaces of first and second jaws and further activates bi-polar electrodes within a surface of at least one jaw. Another mode comprises causing bi-polar electrosurgical energy delivery only between opposing polarity surfaces within a single jaw. Another mode comprises causing bi-polar electrosurgical energy delivery only between opposing polarity surfaces in the first and second jaws. One mode may be configured to substantially dehydrate and reduce the cross-section of an engaged tissue volume. Another method may be configured to weld an engaged tissue volume. Another method may modulate electrosurgical energy to an engaged tissue volume from a positive temperature coefficient material (PTCR) in a jaw surface. Another method may modulate applied energy to an engaged tissue volume from a resistive heating element in a jaw surface that applies energy when Rf paths in tissue are limited due to increased tissue impedance. Another method may modulate applied energy by utilizing a negative temperature coefficient of resistance (NTCR) material in a jaw surface.

In another aspect of the invention, shown by FIGS. 5-6C, the electrosurgical instrument comprises a working end carrying a pair of openable-closeable jaws. At least one of the jaws comprises an energy-delivery surface, the surface comprising an active electrode and a PTCR material. The PTCR material comprises a percentage of the surface of at least 5%, 10%, 25%, 50%, or 75%. Alternatively, at least one of the jaws may comprise an energy-delivery surface comprising an active electrode and a resistive material. The resistive material may comprise a percentage of the surface of at least 5%, 10%, 25%, 50%, or 75%. A resistive material, when intermediate to Rf current paths, will heat up to a selected level and emit heat by conduction after tissue impedance becomes too high. Thus, a suitable tissue temperature may be maintained to assist in welding tissue.

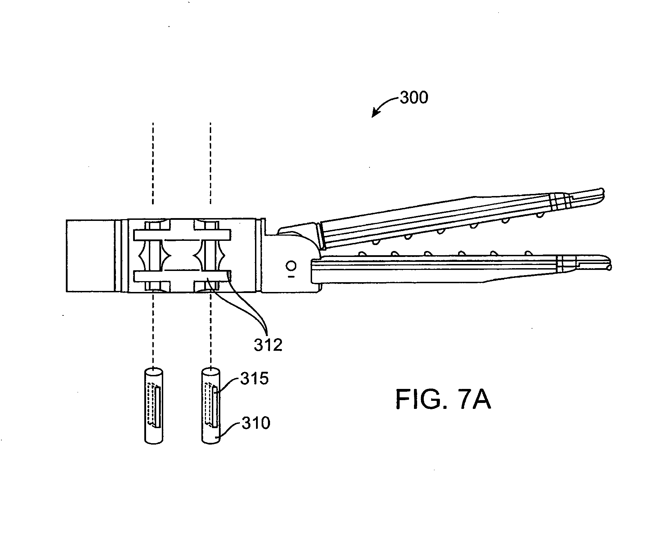

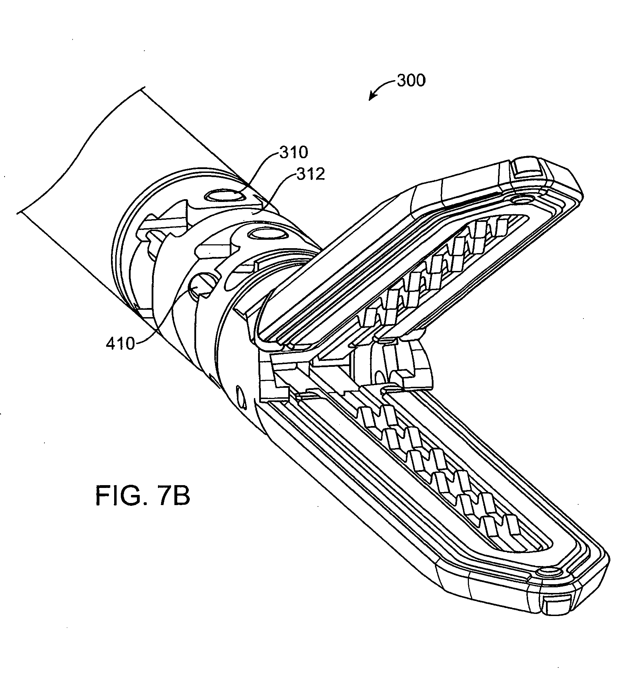

FIG. 7A-B illustrate embodiments of a working end **300** of an electrosurgical instrument. Working end **300** comprises an articulating structure **305** comprising at least one pivotable hinge element that rotate about pins **310.** FIG 7A shows two pivotable elements **310** which allow for movement from a non-deflected configuration to a deflected configuration that deflects that jaws at least 20°, 30°, 40°, 50° or 60°. Each pin **310** that couples the pivotable elements **312** may comprise a slot **315** for receiving the slidable extension member **240** previously described. FIG. 7B is a side-view of the working end of FIG. 7A. Working end **300** may comprise a cable **410** as described below.

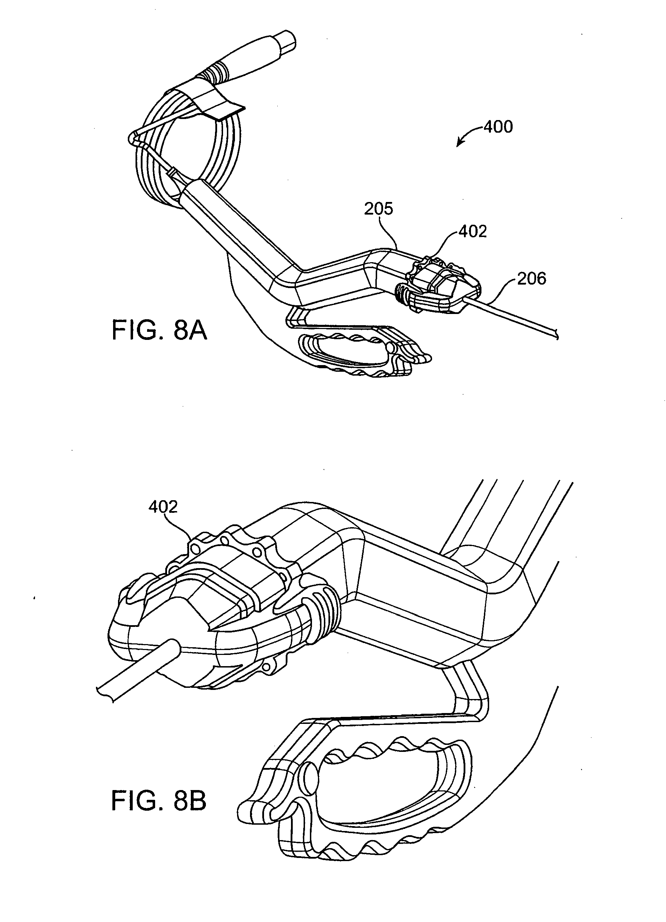

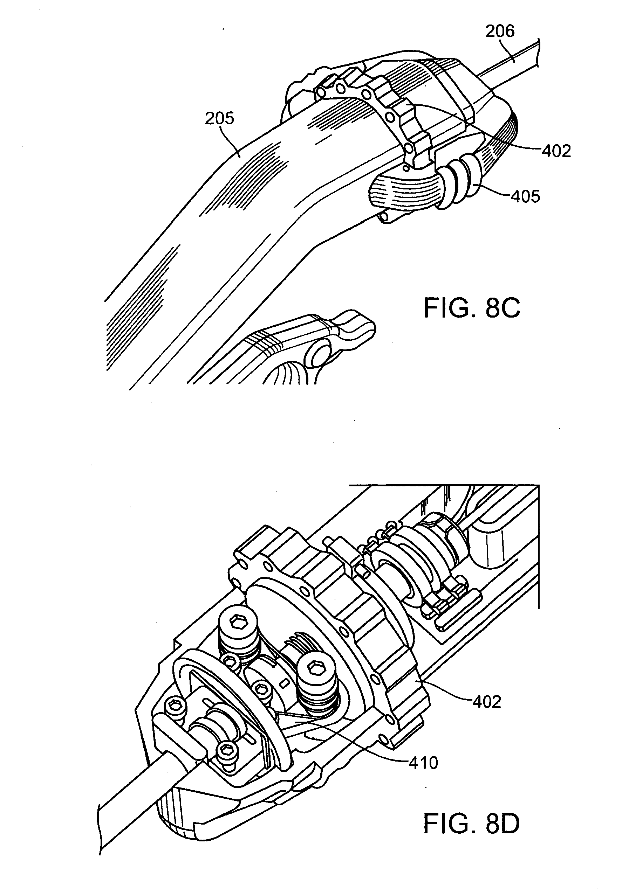

FIGS. 8A-8D illustrate embodiments of electrosurgical instrument **400** which carry the articulating working end **300** of FIG. 7A-B. The handle is configured for in-line actuation for use in vein harvesting procedures. In one embodiment, the handle comprises a thumb-wheel **402** which has dual functions: (i) to rotate the shaft and (ii) to deflect the working end. The thumb-wheel **402** may be switched between the two functions by means of locking mechanism **405.** When locked in the "deflecting" position, actuation of thumb-wheel **302** pulls cables **410** using a well known mechanism in the art for deflecting the end of medical instruments. The jaw closing mechanism comprises a translatable, reciprocating member as previously described.

In should be appreciated that the deflectable working end of FIGS. 8A-8D also can be coupled to multiple actuators or motor drives, for example a surgical robot, to deflect, rotate and close the jaws using only three actuatable mechanisms. The instrument comprises (i) a handle end coupled to a shaft having a working end with a jaw structure, (ii) a jaw-closing mechanism comprising an extension member slidable from a retracted position to an extended position in a longitudinal channel within paired jaws for closing the jaws, the extension member having first surfaces that engage cooperating second surfaces of the paired jaws to move the jaws from an open position to a closed position, (iii) a shaft-rotating mechanism for rotating the shaft and working end relative to the handle end; and (iv) an articulating mechanism for articulating the working end between a non-deflected configuration and a deflected configuration.

Although particular embodiments of the present invention are described above in detail, it will be understood that the description is merely for purposes of illustration. Specific features of the invention are shown in some drawings and may not be shown in others. Any feature may be combined with another in accordance with the embodiments of the invention. Further variations will be apparent to one skilled in the art in light of this disclosure and are intended to fall within the scope of the claims. The invention is set out in the appended claims.

## Claims

1. An electrosurgical instrument (200), comprising:
an instrument body (206);
a working end (210) on the instrument body having a first jaw (222a) and a second jaw (222b), wherein the jaws each have an energy delivery surface (265a; 265b) and are adapted to open and close relative to each other
; and
a control system configured to activate the energy delivery surfaces in a first mode, a second mode, or both based on an operational parameter ;
wherein the first mode of delivering energy substantially dehydrates and reduces the cross-section of the tissue and the second mode of delivering energy welds the tissue; and **characterized in that** the jaws are adapted to open and close relative to each other with a selectable degree of jaw closure between the first and second jaw and the operational parameter comprises the degree of jaw closure.

2. The electrosurgical instrument of claim 1 wherein the control system switches between activating the energy delivery surfaces in the first mode and activating the energy delivery surfaces in the second mode in response to a change in the operational parameter.

3. The electrosurgical instrument of claim 1 wherein the operational parameter further comprises a rate of jaw closure, an impedance of the tissue or a time interval.

4. The electrosurgical instrument of claim 1 wherein at least a portion of the energy delivery surfaces of at least one jaw comprises a resistive heating element and at least a portion of the energy delivery surfaces of at least one jaw comprises a radiofrequency (Rf) element.

5. The electrosurgical instrument of claim 4 wherein the resistive heating element comprises a resistive heating material.

6. The electrosurgical instrument of claim 5 wherein the resistive heating element delivers heat to the tissue when radiofrequency (Rf) paths are limited due to increased tissue impedance.

7. The electrosurgical instrument of claim 5 wherein the resistive heating material extends over at least 5% of the energy delivery surface.

8. The electrosurgical instrument of claim 1 wherein the jaws comprise a positive temperature coefficient of resistance (PTCR) material or a negative temperature coefficient of resistance (NTCR) material.

9. The electrosurgical instrument of claim 1 wherein each of the energy delivery surfaces comprises at least one radiofrequency electrode and the electrodes are arranged to be connected to opposite poles of a bipolar power supply in the control system.

10. The electrosurgical instrument of claim 9 wherein the control system in the first mode activates the electrodes in the surfaces of the first and second jaws, the activated electrodes having a polarity.

11. The electrosurgical instrument of claim 10 wherein the control system in the second mode activates the electrodes in the surfaces of the first and second jaws, the activated electrodes having a polarity opposite of that of the first mode.

12. The electrosurgical instrument of claim 9 wherein the control system in the second mode activates at least one of the electrodes in the surfaces of the first and second jaws and the electrodes within a surface of at least one jaw.

13. The electrosurgical instrument of claim 1 wherein the instrument body comprises:
an axially reciprocating member (240) carried by the instrument body, the reciprocating member configured to open and close the jaws, and
wherein axial movement of the reciprocating member is configured to switch the activation of the electrosurgical surfaces from the first mode to the second mode.

14. The electrosurgical instrument of claim 13 wherein the first jaw and second jaw each comprise a plurality of electrodes and the control system is configured to activate different sets of electrodes based on at least one of the percentage of jaw closure and the impedance of tissue captured between the first and second jaw.

## Patentansprüche

1. Elektrochirurgisches Instrument (200), umfassend:
einen Instrumentenkörper (206);
ein Arbeitsende (210) an dem Instrumentenkörper mit einer ersten Backe (222a) und einer zweiten Backe (222b), worin die Backen jeweils eine Energieabgabefläche (265a; 265b) aufweisen und sich im Verhältnis zueinander öffnen und schließen können; und
ein Kontrollsystem zur Aktivierung Energieabgabeflächen in einem ersten Modus, einem zweiten Modus oder beiden auf Basis eines Betriebsparameters;
worin der erste Modus der Energieabgabe das Gewebe im Wesentlichen dehydriert seinen Querschnitt verringert und der zweite Modus der Energieabgabe das Gewebe verschweißt; und **dadurch gekennzeichnet, dass** sich die Backen im Verhältnis zueinander mit einem wählbaren Backenschließgrad zwischen der ersten und der zweiten Back öffnen und schließen können und der Betriebsparameter den Backenschließgrad umfasst.

2. Elektrochirurgisches Instrument nach Anspruch 1, worin das Kontrollsystem als Reaktion auf eine Veränderung des Betriebsparameters zwischen Aktivierung der Energieabgabeflächen in dem ersten Modus und Aktivierung der Energieabgabeflächen in dem zweiten Modus wechselt.

3. Elektrochirurgisches Instrument nach Anspruch 1, worin der Betriebsparameter ferner eine Backenschließrate, eine Impedanz des Gewebes oder einen Zeitintervall umfasst.

4. Elektrochirurgisches Instrument nach Anspruch 1, worin zumindest ein Teil der Energieabgabeflächen zumindest einer Backe ein Widerstandsheizelement umfasst und zumindest ein Teil der Energieabgabeflächen zumindest einer Backe ein Hochfrequenz (HF)-Element umfasst.

5. Elektrochirurgisches Instrument nach Anspruch 4, worin das Widerstandsheizelement ein Widerstandsheizmaterial umfasst.

6. Elektrochirurgisches Instrument nach Anspruch 5, worin das Widerstandsheizelement Wärme an das Gewebe abgibt, wenn Hochfrequenz (HF)-Wege aufgrund von erhöhter Gewebeimpedanz begrenzt sind.

7. Elektrochirurgisches Instrument nach Anspruch 5, worin sich das Widerstandsheizelement über zumindest 5% der Energieabgabefläche erstreckt.

8. Elektrochirurgisches Instrument nach Anspruch 1, worin die Backen ein Material mit einem positiven Temperaturkoeffizienten des Widerstandes (PTCR) oder ein Material mit einem negativen Temperaturkoeffizienten des Widerstandes (NTCR) umfassen.

9. Elektrochirurgisches Instrument nach Anspruch 1, worin jede Energieabgabefläche zumindest eine Hochfrequenzelektrode umfasst und worin die Elektroden zur Verbindung mit entgegengesetzten Polen einer bipolaren Stromversorgung in dem Kontrollsystem angeordnet sind.

10. Elektrochirurgisches Instrument nach Anspruch 9, worin das Kontrollsystem im ersten Modus die Elektroden in den Oberflächen der ersten und zweiten Backen aktiviert, wobei die aktivierten Elektroden eine Polarität aufweisen.

11. Elektrochirurgisches Instrument nach Anspruch 10, worin das Kontrollsystem im zweiten Modus die Elektroden in den Oberflächen der ersten und zweiten Backen aktiviert, wobei die aktivierten Elektroden eine Polarität aufweisen, die der Polarität des ersten Modus entgegengesetzt ist.

12. Elektrochirurgisches Instrument nach Anspruch 9, worin das Kontrollsystem im zweiten Modus zumindest eine der Elektroden in den Oberflächen der ersten und zweiten Backen und der Elektroden in einer Oberfläche zumindest einer Backe aktiviert.

13. Elektrochirurgisches Instrument nach Anspruch 1, worin der Instrumentenkörper Folgendes umfasst:
ein sich axial hin und her bewegendes Element (240), das von dem Instrumentenkörper getragen wird, wobei das sich hin und her bewegende Element zum Öffnen und
Schließen der Backen ausgelegt ist, und
worin die axiale Bewegung des sich hin und her bewegenden Elements zum Umschalten der Aktivierung der elektrochirurgischen Oberflächen vom ersten Modus in den zweiten Modus ausgelegt ist.

14. Elektrochirurgisches Instrument nach Anspruch 13, worin die erste Backe und die zweite Backe jeweils eine Vielzahl von Elektroden umfassen und das Kontrollsystem zur Aktivierung verschiedener Elektrodensätze auf Basis zumindest der prozentualen Backenschließung und/oder der Impedanz des zwischen den ersten und zweiten Backen festgehaltenen Gewebes ausgelegt ist.

## Revendications

1. Instrument électro-chirurgical (200), comprenant :
un corps d'instrument (206) ;
une extrémité fonctionnelle (210) placée sur le corps d'instrument ayant une première mâchoire (222a) et une seconde mâchoire (222b), dans laquelle les mâchoires ont chacune une surface de répartition d'énergie (265a ; 265b) et sont conçues pour s'ouvrir et se fermer l'une par rapport à l'autre ; et
un système de commande configuré pour activer les surfaces de répartition d'énergie dans un premier mode, un second mode ou les deux modes sur la base d'un paramètre fonctionnel ;
dans lequel le premier mode de répartition d'énergie déshydrate et réduit sensiblement la section transversale du tissu et le second mode de répartition d'énergie soude le tissu ; et
**caractérisé en ce que** les mâchoires sont conçues pour s'ouvrir et se fermer l'une par rapport à l'autre avec un degré de fermeture de mâchoire sélectionnable entre la première et la seconde mâchoire et que le paramètre fonctionnel comprend le degré de fermeture de mâchoire.

2. Instrument électro-chirurgical selon la revendication 1, dans lequel le système de commande bascule entre l'activation des surfaces de répartition d'énergie dans le premier mode et l'activation des surfaces de répartition d'énergie dans le second mode en réponse à un changement du paramètre fonctionnel.

3. Instrument électro-chirurgical selon la revendication 1, dans lequel le paramètre fonctionnel comprend en outre une vitesse de fermeture de mâchoire, une impédance du tissu ou un intervalle de temps.

4. Instrument électro-chirurgical selon la revendication 1, dans lequel au moins une partie des surfaces de répartition d'énergie d'au moins une mâchoire comprend un élément de chauffage par résistance et au moins une partie des surfaces de répartition d'énergie d'au moins une mâchoire comprend un élément de radiofréquence (Rf).

5. Instrument électro-chirurgical selon la revendication 4, dans lequel l'élément de chauffage par résistance comprend un matériau de chauffage par résistance.

6. Instrument électro-chirurgical selon la revendication 5, dans lequel l'élément de chauffage par résistance fournit de la chaleur au tissu lorsque les voies de radiofréquence (Rf) sont limitées par une impédance accrue du tissu.

7. Instrument électro-chirurgical selon la revendication 5, dans lequel le matériau de chauffage par résistance s'étend sur au moins 5 % de la surface de répartition d'énergie.

8. Instrument électro-chirurgical selon la revendication 1, dans lequel les mâchoires comprennent un matériau à coefficient de résistance de température positive (PTCR) ou un matériau à coefficient de résistance de température négative (NTCR).

9. Instrument électro-chirurgical selon la revendication 1, dans lequel chacune des surfaces de répartition d'énergie comprend au moins une électrode à radiofréquence et dans lequel les électrodes sont agencées de façon à relier les pôles opposés d'une alimentation électrique bipolaire dans le système de commande.

10. Instrument électro-chirurgical selon la revendication 9, dans lequel le système de commande active dans le premier mode les électrodes placées dans les surfaces des première et seconde mâchoires, les électrodes activées ayant une polarité.

11. Instrument électro-chirurgical selon la revendication 10, dans lequel le système de commande active dans le second mode les électrodes placées dans les surfaces des première et seconde mâchoires, les électrodes activées ayant une polarité opposée à celle du premier mode.

12. Instrument électro-chirurgical selon la revendication 9, dans lequel le système de commande active dans le second mode au moins une des électrodes placées dans les surfaces des première et seconde mâchoires ainsi que les électrodes placées à l'intérieur d'une surface d'au moins une mâchoire.

13. Instrument électro-chirurgical selon la revendication 1, dans lequel le corps d'instrument comprend :
un élément (240) effectuant un mouvement de va-et-vient axial supporté par le corps d'instrument, l'élément effectuant un mouvement de va-et-vient étant configuré pour ouvrir et fermer les mâchoires ; et
dans lequel le mouvement axial de l'élément effectuant un mouvement de va-et-vient est configuré pour basculer l'activation des surfaces électro-chirurgicales du premier mode dans le second mode.

14. Instrument électro-chirurgical selon la revendication 13, dans lequel la première mâchoire et la seconde mâchoire comprennent chacune une pluralité d'électrodes et dans lequel le système de commande est configuré pour activer différents jeux d'électrodes sur la base d'au moins un élément parmi le pourcentage de fermeture de mâchoire et l'impédance du tissu emprisonné entre la première et la seconde mâchoire.
